# EUROPEAN PATENT APPLICATION

(11) **EP 1 782 781 A1**
(43) Date of publication of application: **09.05.2007**
(21) Application number: 06121911.9
(22) Date of filing: 06.10.2006
(51) Int. Cl.: A61F 9/007, A61M 25/06, A61M 5/32

(54) **Surgical probe**

(30) Priority: 08.11.2005 US 268928
(71) Applicant: Alcon, Inc., 6331 Hünenberg (CH)
(72) Inventor: Auld, Jack R., Laguna Niguel, CA 92677 (US); Zica, Michael A., Costa Mesa, CA 92626 (US); McCollam, Chris, Costa Mesa, CA 92626 (US)
(74) Representative: Hanna, Peter William Derek

(57) **Abstract**

The invention provides a surgical probe (10,110,210) with a thin gauge cannula (14, 114, 214) having a reinforcing sleeve member (16,116,216) adapted to resist bending and being retractably mounted over the cannula, so as to selectively expose a portion of the cannula and protect the remainder of the cannula from bending. The probe is useful in ophthalmic microsurgery.

## Description

### Background of the Invention

This invention relates generally to the field of microsurgery and, more particularly, to ophthalmic microsurgery.

Current vitreoretinal techniques in which surgical instruments are inserted into the eye require the dissection of the conjunctiva and the creation of pars plana scleral incisions through the sclera. The dissection of the conjunctiva typically involves pulling back the conjunctiva about the eye so as to expose large areas of the sclera and the clipping or securing of the conjunctiva in that pulled back state. Following the creation of the incisions, surgical instruments are passed through these incisions and the inserted instruments are observed through the pupil using a microscope and corrective optics. These instruments are used to manipulate and/or dissect retinal tissues within the eye as well as to implement the specific retinal treatment technique (e.g., photocoagulation). Prior art scleral incisions created for vitreoretinal surgery are made large enough to accommodate the required instruments, the inserted portions being typically 19 or 20 gauge (approximately 1 mm) in diameter. After completing the specific treatment procedure, the inserted instruments are removed from the incisions in the sclera. Because the incisions through the sclera are large enough to pass 19 or 20 gauge instruments, the incisions are typically too large to self-seal. Thus, the incisions must be sutured shut. Following the suturing of the scleral incisions, the surgical personnel reposition the conjunctiva in its normal position and reattach the free end(s) of the conjunctiva to the eye using sutures. While such methods and techniques have proven to be effective in the treatment of vitreoretinal disease, there is a strong motivation to move away from procedures requiring sutures and instead look to greatly simplified sutureless procedures. Therefore, recently surgical instruments have been miniaturized so that the cannulas or shafts of the instruments are on the order of 23 or 25 gauge. Such thin shafts are bent easily, particularly as they are manipulated within very tight wounds.

Therefore, a need continues to exist for a thin gauge probe that more easily resists bending during use.

### Brief Summary of the Invention

The present invention improves upon the prior art by providing a thin gauge surgical probe having a retractable reinforcing sleeve.

Accordingly, one objective of the present invention is to provide a thin gauge surgical probe.

Another objective of the present invention is to provide a thin gauge surgical probe that resists bending.

Another objective of the present invention is to provide a thin gauge surgical probe having a retractable reinforcing sleeve.

These and other advantages and objectives of the present invention will become apparent from the detailed description and claims that follow.

### Brief Description of the Drawing

FIG. 1 is a cross-sectional view of a first embodiment of the probe of the present invention.
FIG. 2 is a cross-sectional view of second embodiment of the probe of the present invention.
FIG. 3 is a cross-sectional view of a third embodiment of the probe of the present invention.

### Detailed Description of the Preferred Embodiments

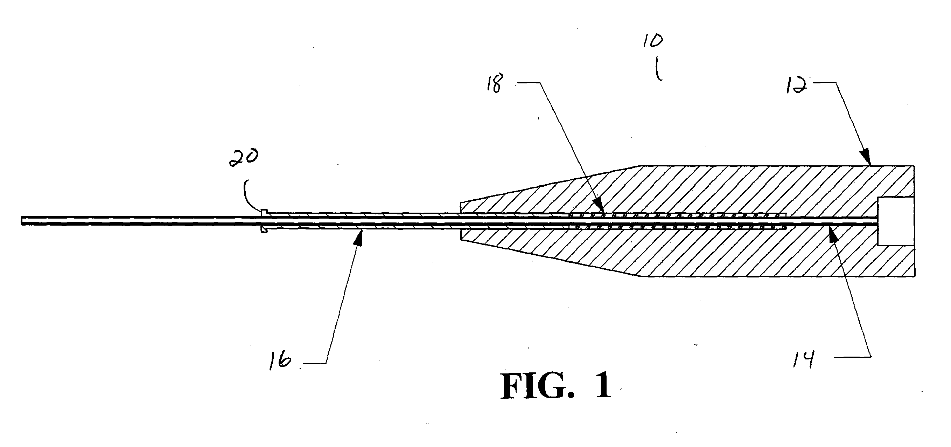

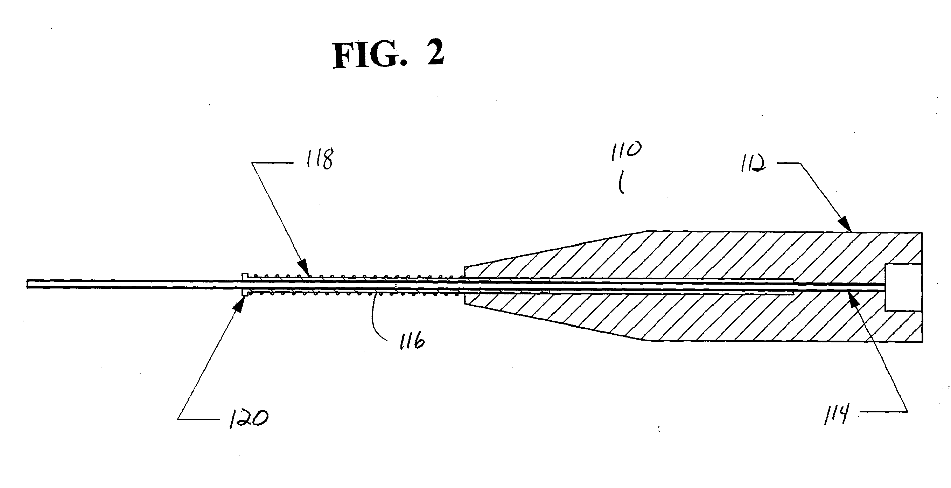

As best seen in FIGS. 1 and 2, probe 10 and 110 consist of probe handle or body 12 and 112 and cannula 14 and 114, respectively. Body 12 and 112 may be made of any suitable material, such as stainless steel, titanium or plastic. Cannula 14 and 114 may be an irrigation/aspiration cannula, or may be the outside cannula for a coaxial cannula system wherein the inner cannula is actuated in some manner, vitrectomy probes, forceps and scissors being examples of the latter. Cannula 14 and 14 generally will be made of thin walled stainless steel or titanium tubing with an outside diameter of 23 or 25 gauge or smaller. Cannula 14 and 114 is joumaled into body 12 or 112 and retained within body 12 or 112 by a frictional fit or an adhesive. As seen in FIG. 1, coaxially mounted over cannula 14 is sliding sleeve 16 and spring 18, with spring 18 being between sleeve 16 and body 12 so that movement of sleeve 16 over cannula 14 toward body 12 causes compression of spring 18. As best seen in FIG. 2, coaxially mounted over cannula 114 is sliding sleeve 116. Spring 118 slides onto sleeve 116, respectively. Spring 18 and 118 biases sleeve 16 and 116 distally along cannula 14 and 114, respectively, when in the relaxed state. When cannula 14 or 114 is inserted into a surgical incision, end cap 20 and 120 on sleeve 16 and 116 contacts the perimeter of the incision so that sleeve 16 and 116 is pushed back toward handle 12 and 112, thereby compressing spring 18 and 118 so that only the portion of cannula 14 or 114 laying outside of the incision as probe 10 and 110 is moved about is covered and supported by sleeve 16 or 116, thereby helping the covered portion of cannula 14 and 114 to resist bending.

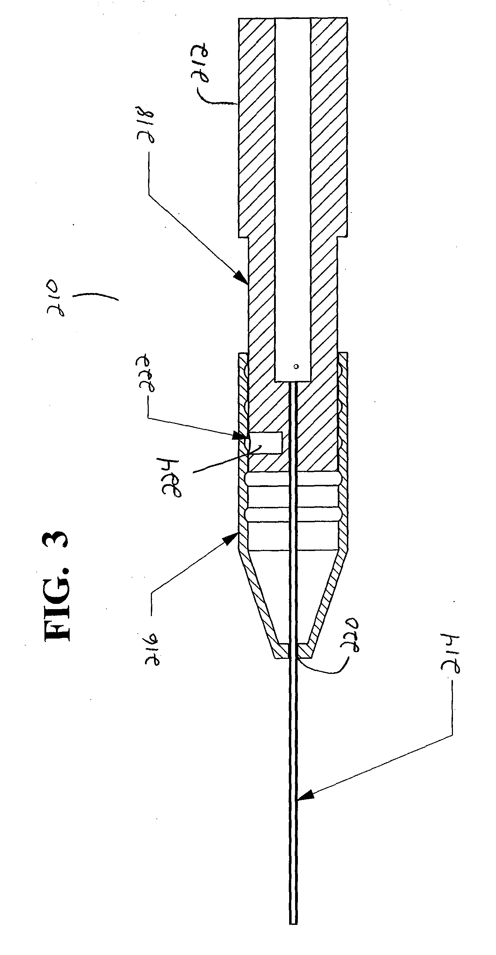

As best seen in FIG. 3, probe 210 of yet another embodiment of the present invention generally include body 212, cannula 214 and slidable nose piece 216. Nose piece 216 is sized to reciprocate over distal end 218 and contains a bore 220 that is received over cannula 214. Nose piece 216 is held in place on body 212 by a plurality of detents 222 on nose piece 216 that interact with locking device 224 on distal end 218 of body 212. The use of a plurality of detents 222 allow nose piece 216 to be locked in multiple positions on distal end 218, each position exposing a different length of cannula 214. Such adjustment allows the surgeon to expose only enough of cannula 214 as may be necessary for the particular probe 210 and surgical procedure and protects the remainder of cannula 214 from bending.

This description is given for purposes of illustration and explanation. It will be apparent to those skilled in the relevant art that changes and modifications may be made to the invention described above without departing from its scope or spirit.

## Claims

1. A surgical probe (10,110,210) comprising:
a body (12,112,212);
a bendable cannula (14,114,214) coaxially mounted to the body;
**characterized by**
a sleeve member (16,116,216) adapted to resist bending being retractably mounted to the body over the cannula, so as to selectively expose a portion of the cannula and protect the remainder of the cannula from bending.

2. The probe (10,110) of claim 1, wherein the sleeve member comprises a sleeve (16,116) slidably received on the cannula, and a spring (18,118) biasing the sleeve to extend from the body.

3. The probe (10,110) of claim 2, wherein the spring (18,118) is coaxially mounted over the sleeve (16,116).

4. The probe (10,110) of claim 2, wherein the spring (18,118) is coaxially mounted on the cannula (14,114).

5. The probe (10,110) of any one of claims 2 to 4, wherein the sleeve further comprises an end cap or flange (20), adapted to prevent the sleeve from entering a surgical incision.

6. The probe (210) of claim 1, wherein the body (212) defines a locking device (224), and wherein the sleeve member comprises a nose piece (216) coaxially mounted over the cannula and the body, the nose piece having a plurality of spaced detents (222) adapted to interact with the locking device, so as to fix the nose piece in one of a plurality of extended positions with respect to the body.

7. The probe (10,110,210) of any one of the preceding claims, wherein the cannula is of 23 or 25 gauge or smaller.
